# EUROPÄISCHE PATENTSCHRIFT

(11) **EP 2 753 374 B1**
(45) Veröffentlichungstag und Bekanntmachung des Hinweises auf die Patenterteilung: **12.10.2016**
(21) Anmeldenummer: 12743165.8
(22) Anmeldetag: 02.08.2012
(51) Int. Cl.: A61M 1/00

(54) **WUNDAUFLAGE ZUR VERWENDUNG BEI DER UNTERDRUCKBEHANDLUNG VON WUNDEN**
WOUND DRESSING FOR USE IN NEGATIVE-PRESSURE WOUND THERAPY
PANSEMENT À UTILISER POUR LE TRAITEMENT DE PLAIES PAR PRESSION NÉGATIVE

(30) Priorität: 08.09.2011 DE 102011082347
(43) Veröffentlichungstag der Anmeldung: 16.07.2014
(73) Patentinhaber: Paul Hartmann AG, 89522 Heidenheim (DE)
(72) Erfinder: CROIZAT, Pierre, 89542 Herbrechtingen (DE); ECKSTEIN, Axel, Heidenheim 89522 (DE)
(74) Vertreter: DREISS Patentanwälte PartG mbB
(86) Internationale Anmeldenummer: PCT/EP2012/065130
(87) Internationale Veröffentlichungsnummer: WO 2013/034373

(56) Entgegenhaltungen:
- WO-A2-03/057071
- US-A1- 2008 287 892
- US-A1- 2010 268 128

## Beschreibung

Die Erfindung betrifft eine Sachgesamtheit nach dem Oberbegriff des Anspruchs 1. Wundauflagen zur Verwendung bei der Unterdruckbehandlung von Wunden sind beispielsweise in WO 2004/037334 und WO 2010/033272 vorbeschrieben. Eine nicht gattungsgemäße Sachgesamtheit für die Unterdruckbehandlung von Wunden ist bekannt aus WO 03/057071 A2. Bei dieser Sachgesamtheit ist ein Absaugleitung, die auch als Zuführleitung für Irrigationsflüssigkeit dienen kann, unterhalb des Unterdruckverbands in einen Wundraum geführt und dort mit einer Anschlussvorrichtung verbunden, über welche der Unterdruck oder die lrrigationsflüssigkeit im Bereich des Wundgrunds verteilt wird.

US 2008/0287892 A1 zeigt eine Sachgesamtheit für die Unterdruckbehandlung von Wunden, bei deren hülsenförmiges Trennmittel um eine Fistelöffnung herum angeordnet ist und zum Abführen von aus der Fistelöffnung austretenden Körperflüssigkeiten mit einer Ostomievorrichtung zusammenwirkt.

In der jüngeren Vergangenheit hat die Unterdruckbehandlung von Wunden, insbesondere von tiefen und daher a priori problematisch heilenden Wunden, eine zunehmende Bedeutung erlangt. Dabei bedeutet Unterdruckbehandlung, dass ein an sich der umgebenden Atmosphäre ausgesetzter Körper- oder Wundbereich durch noch näher zu beschreibende Mittel druckdicht bzw. unterdruckdicht gegen die Umgebung, also die Atmosphäre in der wir leben und atmen, abgeschlossen wird, wobei innerhalb des abgeschlossenen Wundbereichs auf ebenfalls noch zu erläuternde Weise ein gegenüber dem Atmosphärendruck verringerter Druck, mithin also Unterdruck relativ zur Atmosphäre angelegt und dauerhaft aufrechterhalten werden kann. Wenn auf dem hier in Rede stehenden Gebiet von Unterdruck die Rede ist, so wird hiermit ein Druckbereich verstanden, der typischerweise zwischen 0 und 250 mmHg (mm Quecksilbersäule) unterhalb des umgebenden Atmosphärendrucks liegt. Es hat sich gezeigt, dass dies der Wundheilung förderlich ist. Für den unterdruckdichten Abschluss wird ein Unterdruckverband vorgesehen, der beispielsweise eine druck- bzw. unterdruckdichte Folienschicht umfassen kann, die typischerweise mit einem die Wunde umgebenden unversehrten Körperbereich verklebt ist, so dass auf diese Weise ein dichter Abschluss erreicht werden kann. Um ausgehend von einer Unterdruck erzeugenden Einrichtung, also einer Unterdruckpumpe im weitesten Sinn, einen Unterdruck an den Wundraum heranzuführen und dort aufrechtzuerhalten, können bei hier in Rede stehenden Systemen zur Unterdrucktherapie von Wunden mit Unterdruck beaufschlagbare Leitungsmittel verwendet werden, die mittels einer Anschlussvorrichtung mit dem Unterdruckverband zusammenwirken, um Unterdruck an bzw. in den Wundraum zu bringen.

Der vorliegenden Erfindung liegt die Aufgabe zugrunde, eine Sachgesamtheit der eingangs genannten Art dahingehend zu verbessern, dass sie sich vielfältiger, insbesondere für eine Instillationstherapie, einsetzen lässt.

Diese Aufgabe wird erfindungsgemäß durch eine Sachgesamtheit mit den Merkmalen des Anspruchs 1 gelöst.

Dadurch, dass in der Durchgangsöffnung der schaumbasierten Wundauflage erfindungsgemäß eine Hülse angeordnet ist, lässt sich ein einfaches und sicheres Einfügen des wundseitigen Endabschnitts der Fluidzuführleitung realisieren. Hierdurch erhält dieser Endabschnitt eine vorgegebene Orientierung im Wundraum, ohne dass die behandelnde Person beim Applizieren der Komponenten für die Unterdruckbehandlung mit Leitungskomponenten im Wundraum oder innerhalb der Wundauflage aufwändig hantieren muss, um eine intendierte Anordnung zu erreichen. Außerdem besteht in geringerem Maße die Gefahr, dass es beim Anordnen der Komponenten zu schmerzauslösenden Beeinträchtigungen beim Patienten kommt. Es erweist sich insoweit auch als besonders vorteilhaft, dass durch die erfindungsgemäße Anordnung der Hülse der besagte Endabschnitt der Fluidzuführleitung nicht über die dem Wundgrund zugewandte Seite der Wundauflage vorzustehen braucht, so dass eine Reizung des Wundgrunds infolge der Fluidzuführleitung vermieden werden kann. Dennoch kann erfindungsgemäß sichergestellt werden, dass über die Fluidzuführleitung zugeführte flüssige oder gasförmige Medien, wie z.B. Spülflüssigkeit oder Sauerstoff, bis zum Wundgrund geführt werden, wo sie in besonderem Maße benötigt werden. Wenn nämlich beispielsweise Spülflüssigkeit oder Flüssigkeit mit pharmazeutisch wirksamen und die Wundheilung unterstützenden Wirkstoffen lediglich in einen vom Wundgrund abgewandten Bereich der Wundauflage eingeleitet und gleich wieder über eine Saugleitung abgesaugt wird, so ist die therapeutische Wirkung dieser Maßnahme nur sehr reduziert. Mit der erfindungsgemäßen Wundauflage wird hingegen sichergestellt, dass zugeführtes Fluid bis zum Wundgrund gelangen und dort seine intendierte therapeutische Wirkung ausüben kann. Auch wenn lediglich Spülflüssigkeit, beispielsweise in Form einer Ringerlösung, zugeführt wird, kann hierdurch eine effektive Spülung und damit Reinigung des Wundraums erreicht werden.

Die Hülse liegt vorzugsweise direkt gegen das Schaummaterial der Wundauflage an.

In weiterer Ausbildung der Erfindung erweist es sich als vorteilhaft, wenn die Hülse wenigstens auf einer Seite, vorzugsweise auf beiden Seiten des Schaummaterials einen Bundabschnitt aufweist, mit dem die Hülse bezüglich einer Hülsenlängsachse axial gegen eine die Öffnung umgebende Seite des Schaummaterials anliegt. Hierdurch kann ein bündiger und von Kanten freier Übergang von der Hülse zum Schaummaterial der Wundauflage erreicht werden, und das Einführen des Endabschnitts der Fluidzuführleitung gestaltet sich einfacher. Weiter erweist es sich als vorteilhaft, wenn der Bundabschnitt in Umfangsrichtung durchgehend geschlossen ausgebildet ist.

Nach einem weiteren Erfindungsgedanken von besonderer Bedeutung erweist es sich als vorteilhaft, wenn die Länge der Hülse veränderbar ist und sich die Hülse so der Kompression oder Kontraktion des Schaummaterials der Wundauflage anpassen kann. Hierfür kann die Hülse nach einem weiteren Erfindungsgedanken zwei gegeneinander in Richtung einer Hülsenlängsachse gleitverschiebliche, teleskopierende Hülsenabschnitte umfassen. Nach einem anderen Erfindungsgedanken wäre es denkbar, dass die Hülse zwei über eine faltenbalgartige Kopplung in Richtung der Hülsenlängsachse gegeneinander bewegbare Hülsenabschnitte umfasst.

Die Hülse kann beispielsweise aus Kunststoffen, wie Thermoplasten, Elastomeren, thermoplastischen Elastomeren oder Silikon, gebildet sein.

Die Wundauflage umfasst ein flexibel nachgiebiges Schaummaterial, wobei es sich bei dem Schaummaterial vorzugsweise um einen offenzelligen Polyurethanschaumstoff (PUR-Schaumstoff) handelt. Als Schaumstoffe werden üblicherweise Werkstoffe mit über die gesamte Masse verteilten Zellen (offen, geschlossen oder beides) verstanden. Solche Werkstoffe weisen üblicherweise somit eine Rohdichte (gemäß DIN EN ISO 845) auf, die niedriger ist als die Dichte der Gerüstsubstanz.

### [Zelle]

Eine Zelle ist der bei der Herstellung von Schaumstoffen gebildete einzelne Hohlraum, der von Zellwänden und/oder Zellstegen teilweise oder vollständig umschlossen ist.

### [Offenzellig]

Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht. Im Rahmen dieser Anmeldung bedeutet der Begriff offenzellig, dass im Polyurethanschaumstoff mindestens 60 % offene Zellen, bevorzugt mindestens 90 % offene Zellen, mehr bevorzugt mindestens 98 % offene Zellen, insbesondere im Wesentlichen 100 % offene Zellen, bezogen auf die Gesamtzahl an Zellen, vorhanden sind. Die Offenzelligkeit des Polyurethanschaumstoffs wird üblicherweise nach ASTM D 2856-87, Verfahren B) bestimmt.

### [Zugfestigkeit]

Gemäß der Erfindung weist der Polyurethanschaumstoff üblicherweise eine Zugfestigkeit nach dreitägiger Lagerung in Rinderserum zwischen 80 kPa und 300 kPa, bevorzugt zwischen 110 kPa bis 250 kPa, mehr bevorzugt zwischen 120 kPa bis 230 kPa, noch mehr bevorzugt von 130 bis 220 kPa, besonders bevorzugt von 140 bis 200 kPa, ganz besonders bevorzugt von 155 bis 190 kPa und insbesondere von 160 bis 185 kPa, auf.

Rinderserum ist an sich im Fachgebiet bekannt. Hierbei handelt es sich um ein Serum, das von Rinderblut gewonnen wird. Bevorzugt wird das unter dem Handelsnamen HyClone® "Standard Fetal Bovine Serum" vertriebene Rinderserum der Firma Thermo Scientific verwendet. In einer bevorzugten Ausführungsform weist das verwendete Rinderserum im Wesentlichen folgende Zusammensetzung bzw. Eigenschaften auf:

**Tabelle 1**

| **Proteingehalt und weitere Werte** | | |
|---|---|---|
| Albumin | 1,9 | gm/dl |
| Alkalische Phosphatase | 213 | mU/ml |
| Blut-Harnstoff Stickstoff | 12 | mg/dl |
| Kreatinin | 2,77 | mg/dl |
| Gammaglobulin | 1,7 | % tp |
| Blutzucker (Glucose) | 107 | mg/dl |
| Glutamat-Oxalacetat-Transaminase (SGOT) | 152 | mU/ml |
| Glutamat-Pyruvat-Transaminase (SGPT) | 37 | mU/ml |
| IgG - Nephelometer | 0,14 | mg/ml |
| Lactatdehydrogenase | 2479 | mU/ml |
| Osmolalität | 312 | mOsm/kg |
| pH | 7,18 | |
| Gesamtbilirubin | 0,4 | mg/dl |
| Gesamtprotein | 3,7 | gm/dl |

| **Gehalt Spurenelement und Eisen** | | |
|---|---|---|
| Calcium | 13,1 | mg/dl |
| Chlorid | 99 | mEq/l |
| Anorganischer Phosphor | 9,6 | mg/dl |
| Eisen | 160 | ug/dl |
| Sättigungskonzentration (Eisen) | 79 | % |
| Kalium | >10,0 | mEq/l |
| Natrium | 133 | mEq/l |
| Gesamt-Eisenbindungskapazität (TIBC) | 201 | ug/dl |

Die zu vermessende Probe wird für 3 Tage bei 23 °C in Rinderserum eingelegt und untergetaucht. Anschließend wird die Zugfestigkeit gemäß DIN 53571 bestimmt. Im Rahmen dieser Anmeldung bedeutet der Ausdruck "gemäß DIN 53571", dass die Bestimmung der Zugfestigkeit grundsätzlich gemäß dieser Norm erfolgt, wobei jedoch in Abweichung zur Norm die 3 Tage in Rinderserum gelagerte Probe nicht vollständig getrocknet wird. Stattdessen wird die Probe aus dem Rinderserum entnommen und in 1 Liter Wasser zum Spülen getaucht. Anschließend wird der Probekörper mit Zellstoffpapier ausgedrückt. Zudem wird in Abweichung zur Norm ein rechteckiger Probekörper mit den Abmessungen 10 x 12,5 x 75 verwendet.

Die Messung der Zugfestigkeit erfolgt mit einem Zugprüfgerät nach EN ISO 527-1 [April 1996] der Firma Zwick (Ulm). Folgende Prüfparameter werden verwendet:
Prüfgeschwindigkeit: 500 mm/min
Einspannlänge: 50 mm
Vorkraft: 0,1 N
Probenbreite b0: 12,5 mm.
[Bruchdehnung]

Ferner weist der Polyurethanschaumstoff bevorzugt eine Bruchdehnung von 150 % bis 700 %, mehr bevorzugt von 200 % bis 650 %, noch mehr bevorzugt von 240 % bis 340 %, insbesondere 260 bis 320 %, gemessen gemäß DIN 53571 (Verfahren 1, Probekörper A), auf. Zudem weist der Polyurethanschaumstoff bevorzugt eine Härte von 20 bis 70 Shore A, mehr bevorzugt von 30 bis 60 Shore A, noch mehr bevorzugt von 40 bis 50 Shore A, gemessen gemäß DIN 53505, auf, wobei die Messung bei 23 °C an einem plattenförmigen, ebenen und glatten Probekörper einer Dicke von 6 mm erfolgte.

### [Luftdurchlässigkeit]

Es hat sich ferner gezeigt, dass vorstehend genannte Aufgaben unerwartet vorteilhaft gelöst werden können, wenn der Polyurethanschaumstoff eine spezielle Luftdurchlässigkeit aufweist. In einer bevorzugten Ausführungsform weist der Polyurethanschaumstoff eine Luftdurchlässigkeit von 1000 bis 8000 l/(m²sec), mehr bevorzugt von 1500 bis 6000 l/(m²see), noch mehr bevorzugt von 2000 bis 5000 l/(m²sec), besonders bevorzugt von 2300 bis 4000 l/(m²sec) und insbesondere von 2400 bis 3300 l/(m²sec), gemessen gemäß DIN EN ISO 9237 (20 mm Prüfdicke, 20 cm² Prüffläche, 200 Pa Differenzdruck) auf.

### [Viskoelastisches Verhalten]

Es hat sich weiterhin gezeigt, dass vorstehend genannte Aufgaben unerwartet vorteilhaft gelöst werden können, wenn der Polyurethanschaumstoff viskoelastisches Verhalten zeigt. Hierunter wird verstanden, dass das Verhalten des Polyurethanschaumstoffs auf Beanspruchung so aussieht, wie die Kombination aus einem elastischen Feststoff und einer viskosen Flüssigkeit. Das viskoelastische Verhalten kann durch einen Torsionsschwingungsversuch gemäß DIN 53445, Verfahren A, charakterisiert werden. Es ist bevorzugt, dass der Schaumstoff bei der Bestimmung gemäß DIN 53445, Verfahren A bei 23 °C einen mechanischen Verlustfaktor von 0,1 bis 1,0, mehr bevorzugt von 0,15 bis 0,8, noch mehr bevorzugt von 0,2 bis 0,6, aufweist.

### [Rohdichte]

Es hat sich weiterhin gezeigt, dass vorstehend genannte Aufgaben unerwartet vorteilhaft gelöst werden können, wenn der Schaumstoff eine Rohdichte zwischen 15 und 55 kg/m³, mehr bevorzugt zwischen 20 und 35 kg/m³, noch mehr bevorzugt zwischen 22 und 30 kg/m³, insbesondere zwischen 24 und 28 kg/m³, aufweist, gemessen gemäß DIN EN ISO 845 (Probekörper mit Abmessungen 100 mm x 100 mm x 50 mm, Konditionierung für 24 h im Normklima (23°C, 50 % rel. Luftfeuchte, 1013 mbar)).

Sofern in den einschlägigen Normen nichts anderes angegeben, werden im Allgemeinen alle Testmethoden bei 23°C, 50 % rel. Luftfeuchte und 1013 mbar Druck durchgeführt.

Schaumstoff - Chemische Zusammensetzung:
Bevorzugte Ausführungsformen der einsetzbaren Polyurethanschaumstoffe (PUR) werden nachstehend erläutert. Der Potyurethanschaumstoff ist üblicherweise erhältlich durch Umsetzung einer Mischung, umfassend die Komponenten
   (i-PUR) Polyisocyanat,
   (ii-PUR) gegenüber Isocyanat reaktive Verbindungen, insbesondere Polyol,
   (iii-PUR), Treibmittel,
   (iv-PUR), Katalysator und
   (v-PUR) gegebenenfalls Zusatzstoffe.

Als Isocyanate (i-PUR) können allgemein bekannte aliphatische, cycloaliphatische und/oder insbesondere aromatische Polyisocyanate eingesetzt werden. Zur Herstellung der Polyurethane eignen sich beispielsweise Diphenylmethandiisocyanat (MDI), hier insbesondere 4,4'-Diphenylmethandiisocyanat (4,4'-MDI), Mischungen aus monomeren Diphenylmethandiisocyanaten und höherkemigen Homologen des Diphenylmethandiisocyanats (PMDI), Tetramethytendiisocyanat (TMDI), Hexamethylendiisocyanat (HDI), Toluylendiisocyanat (TDI) oder Mischungen daraus.

Bevorzugt wird MDI, insbesondere 4,4'-MDI und/oder HDI verwendet. Das besonders bevorzugt verwendete 4,4'-MDI kann geringe Mengen, bis etwa 10 Gew.-%, allophanat- oder uretonimin-modifizierte Polyisocyanate enthalten. Es können auch geringe Mengen Polyphenylenpolymethylenpolyisocyanat (PMDI) eingesetzt werden. Die Gesamtmenge dieser PMDI sollte 5 Gew.% des eingesetzten Isocyanats nicht überschreiten.

Die Polyisocyanatkomponente (i-PUR) wird bevorzugt in Form von Polyisocyanatprepolymere eingesetzt. Diese Polyisocyanatprepolymere sind erhältlich, indem vorstehend beschriebene Polyisocyanate (i-PUR), beispielsweise bei Temperaturen von 30 bis 100 °C, bevorzugt bei etwa 80 °C mit einem Unterschuss an nachstehend beschriebenen Polyolen (ii-PUR) zum Prepolymer umgesetzt werden. Das Polyol-Polyisocyanat-Verhältnis wird hierbei so gewählt, dass der NCO-Gehalt des Prepolymeren 8 bis 28 Gew. -%, vorzugsweise 14 bis 26 Gew. -%, besonders bevorzugt 17 bis 23 Gew. -%, beträgt.

Als gegenüber Isocyanaten reaktive Verbindungen (ii-PUR) werden üblicherweise Polyole wie Polyetherole und/oder Polyesterole verwendet.

Möglich sind Polyetherpolyalkohole (in dieser Anmeldung als "Polyetherpolyole" bezeichnet) mit einer OH-Funktionalität von 1,9 bis 8,0, einer Hydroxylzahl von 50 bis 1000 mg KOH/g sowie gegebenenfalls 10 bis 100 % primären Hydroxylgruppen. Derartige Polyetherpolyole sind bekannt, kommerziell erhältlich, und basieren beispielsweise auf Starterverbindungen, die mit Alkylenoxiden, beispielsweise Propylenoxid und/oder Ethylenoxid, unter allgemein bekannten Bedingungen umgesetzt werden. Der Gehalt an primären Hydroxylgruppen kann erreicht werden, indem man die Polyole zum Abschluss mit Ethylenoxid umsetzt. Bevorzugt werden bei der Herstellung des offenzelligen Schaumstoffs (c) keine Polyetherpolyole verwendet.

Bevorzugt werden Polyesterpolyole in der Komponente (ii-PUR) verwendet. Die verwendeten Polyesterole (ii-PUR) werden im allgemeinen durch Kondensation von mehrfunktionellen Alkoholen, vorzugsweise Diolen, mit 2 bis 12 Kohlenstoffatomen, vorzugsweise 2 bis 6 Kohlenstoffatomen, mit mehrfunktionellen Carbonsäuren mit 2 bis 12 Kohlenstoffatomen, bevorzugt 4 bis 8 Kohlenstoffatomen hergestellt. Beispiele für geeignete Säuren sind Bernsteinsäure, Glutarsäure, Adipinsäure, Phthalsäure, Isophthalsäure, und/oder Terephthalsäure und Gemischen hiervon, hergestellt. Adipinsäure ist besonders bevorzugt. Beispiele für geeignete zwei- und mehrwertige Alkohole sind Ethandiol, Diethylenglykol, 1,4-Butandiol, 1,5-Pentandiol, und/oder 1,6-Hexandiol und Gemische hiervon. 1,4-Butandiol ist besonders bevorzugt.

Die Umsetzungsbedingungen von Carbonsäure und Alkohol werden üblicherweise so gewählt, dass die resultierenden Polyesterole keine freien Säuregruppen aufweisen. Ferner weisen die resultierenden Polyesterole im allgemeinen ein gewichtsmittleres Molekulargewicht (bestimmt mittels Gelpermeationschromatographie) von 500 bis 3500 g/mol, bevorzugt von mehr als 1000 g/mol bis 3000 g/mol, insbesondere von 1500 bis 2500 g/mol, auf. Im allgemeinen weisen die eingesetzten Polyesterole eine mittlere theoretische Funktionalität von 2,0 bis 4, gegebenenfalls von mehr als 2 bis weniger als 3, auf. Des Weiteren weisen im allgemeinen die eingesetzten Polyesterole eine mittlere OH-Zahl von 20 bis 150, bevorzugt von 30 bis 80, auf.

In einer bevorzugten Ausführungsform weisen die verwendeten Polyesterole eine Viskosität von 150 mPa·s bis 600 mPa·s, bevorzugt von 200 mPa·s bis 550 mPa·s, mehr bevorzugt von 220 mPa·s bis 500 mPa·s, besonders bevorzugt von 250 mPa·s bis 450 mPa·s und insbesondere von 270 mPa·s bis 350 mPa·s, gemessen nach DIN 53 015 bei 75 °C, auf.

Die Verbindungen (ii-PUR) können in Mischung mit Kettenverlängerungs- und/oder Vernetzungsmitteln verwendet werden. Bei den Kettenverlängerungsmitteln handelt es sich überwiegend um 2-funktionelle Alkohole mit Molekulargewichten von 60 bis 499, beispielsweise Ethylenglykol, Propylenglykol, Butandiol-1,4, Pentandiol-1,5, Dipropylenglykol und/oder Tripropylenglykol. Bei den Vernetzungsmitteln handelt es sich um Verbindungen mit Molekulargewichten von 60 bis 499 und 3 oder mehr aktiven H-Atomen, vorzugsweise Aminen, und besonders bevorzugt Alkoholen, beispielsweise Glyzerin, Trimethylolpropan und/öder Pentaerythrit.

In einer bevorzugten Ausführungsform enthält (oder besteht aus) die Komponente (ii-PUR) 0-25 Gew.-%, bevorzugt 1 bis 20 Gew.-%, Kettenverlängerungs- und/oder Vernetzungsmittel und 75 bis 100 Gew.-%, bevorzugt 80 bis 99 Gew.-% Polyole(n), insbesondere Polyesterpolyole(n), bezogen auf das Gesamtgewicht der Komponente (ii-PUR)

Als Katalysatoren (iii-PUR) können Verbindungen eingesetzt werden, welche die Reaktion der Komponente (i-PUR) mit der Komponente (ii-PUR) beschleunigen. In Frage kommen beispielsweise tertiäre Amine und/oder organische Metallverbindungen, insbesondere Zinnverbindungen. Beispielsweise können als Katalysatoren folgende Verbindungen eingesetzt werden: Triethylendiamin, Aminoalkyl- und/oder Aminophenylimidazole und/oder Zinn-(II)salze von organischen Carbonsäuren. Katalysatoren werden im allgemeinen in einer Menge von 0,1 bis 5 Gew.-% bezogen auf das Gewicht der Komponente (ii-PUR) eingesetzt.

Als Treibmittel (iv-PUR) können allgemein bekannte chemisch oder physikalisch wirkende Verbindungen eingesetzt werden. Als physikalisch wirkendes Treibmittel kann bevorzugt Wasser eingesetzt werden, welches durch Reaktion mit den Isocyanatgruppen Kohlendioxid bildet. Beispiele für physikalische Treibmittel sind (cyclo)aliphatische Köhlenwasserstoffe, vorzugsweise solche mit 4 bis 8, besonders bevorzugt 4 bis 6 und insbesondere 5 Kohlenstoffatomen, teilhalogenierte Kohlenwasserstoffe oder Ether, Ketone oder Acetate. Die Menge der eingesetzten Treibmittel richtet sich nach der angestrebten Dichte der Schaumstoffe. Die unterschiedlichen Treibmittel können einzeln oder in beliebigen Mischungen untereinander zum Einsatz kommen. Besonders bevorzugt wird nur Wasser als Treibmittel eingesetzt, im allgemeinen in einer Menge von 0,1 bis 5 Gew.-%, insbesondere von 2,5 bis 4 Gew.-%, bezogen auf das Gewicht der Komponente (ii-PUR). Physikalische Treibmittel werden bevorzugt in einer Menge von < 0,5 Gew.-%, bezogen auf das Gewicht der Komponente (ii-PUR), eingesetzt.

Zur Herstellung von Polyurethanschaumstoffen werden im allgemeinen die Komponenten (i-PUR) und (ii-PUR) in solchen Mengen zur Umsetzung gebracht, dass das Äquivalenzverhältnis von NCO-Gruppen zur Summe der reaktiven Wasserstoffatome (insbesondere zu Summe der OH-Gruppen) 1 :0,8 bis 1:1,25, vorzugsweise 1 :0,9 bis 1 :1,15, beträgt. Ein Verhältnis von 1:1 entspricht hierbei einem NCO-Index von 100. Die gewünschte Offenzelligkeit des Polyurethanschaums wird im allgemeinen durch eine dem Fachmann bekannte geeignete Wahl der Komponenten (i-PUR) bis (iv-PUR) gewährleistet. Gegebenenfalls wird nach der Aushärtung der resultierende PUR-Schaumstoff retikuliert.

Die Umsetzung erfolgt gegebenenfalls in Anwesenheit von (v-PUR) Hilfs- und/oder Zusatzstoffen, wie z. B. Füllstoffen, Zellreglem, Zellöffnem, oberflächenaktiven Verbindungen und/oder Stabilisatoren gegen oxidativen, thermischen oder mikrobiellen Abbau oder Alterung.

Es hat sich weiterhin gezeigt, dass vorstehend genannte Aufgaben unerwartet vorteilhaft gelöst werden können, wenn der Polyurethanschaumstoff (c) Silber in Form von Silberionen oder in Form von atomarem Silber enthält. Bevorzugt wird nach der Herstellung des Polyurethanschaumstoffs eine Silberbeschichtung aufgebracht. Alternativ kann das Silber bereits in die härtbare Zusammensetzung miteingebracht werden. Bevorzugt enthält der Polyurethanschaumstoff 0,000001 bis 0,1 Gew.-%, mehr bevorzugt 0,0001 bis 0,01 Gew.-% Silber, bezogen auf das Gesamtgewicht des Polyurethanschaumstoffs.

Es hat sich ferner gezeigt, dass die eingangs beschriebenen Aufgaben mit Polyurethanen auf Basis von lediglich aliphatischen Ausgangsstoffen nicht immer befriedigend gelöst werden könnten. Vielmehr wirkt sich die Verwendung von aromatischen Aufbaukomponenten (i-PUR und/oder ii-PUR) vorteilhaft aus. In einer bevorzugten Ausführungsform weist deshalb der Polyurethanschaumstoff (c) einen Aromatengehalt von 5 bis 50 %, mehr bevorzugt von 10 bis 45 %, insbesondere von 15 bis 40 % auf. Der Aromatengehalt wird bestimmt durch das Verhältnis Gewicht an aromatischen Ringen zu Gesamtgewicht des Schaumstoffs.

Die vorliegende Erfindung betrifft also eine Sachgesamtheit aus einer Wundauflage und aus einem den Wundraum im Wesentlichen unterdruckdicht abschließenden Wundverband und aus einer Anschlussvorrichtung für eine Saugleitung mit den Merkmalen der Ansprüche 1 bis 14.

Es wird also erfindungsgemäß vorgeschlagen, eine zusätzliche Fluidzuführleitung vorzusehen, welche sich durch die Anschlussvorrichtung hindurch erstreckt, derart, dass auf der wundzugewandten Seite des Trägermittels ein Abschnitt der Fluidzuführleitung vorhanden ist, der zur direkten Einleitung in den Wundraum bzw. in die Hülse der dort vorgesehenen Wundauflage genutzt werden kann, um so eine Fluideinleitung direkt in den Wundraum zu realisieren. Auf diese Weise lässt sich die Anschlussvorrichtung zu einer Unterdruckbehandlung einsetzen, im Zuge derer kontinuierlich oder diskontinuierlich ein Fluid, insbesondere eine Flüssigkeit oder ein Gas, wie z.B. Sauerstoff, insbesondere mit pharmazeutisch wirksamen und die Wundheilung unterstützenden Wirkstoffen, dem Wundraum zugeführt werden kann. Die Anschlussvorrichtung kann also zur sogenannten Instillationstherapie eingesetzt werden. Das über die Fluidzuführleitung zuführbare Fluid kann auch aritimikrobiell oder analgetisch wirken. Es kann sich hierbei aber auch lediglich um eine Spüllösung, wie insbesondere eine Ringerlösung, handeln. Dadurch, dass die Fluidzuführleitung erst unterhalb-des Trägermittels in den Wundraum mündet, kann sichergestellt werden, dass die zuzuführende Flüssigkeit auch wirklich in den Wundraum und vorzugsweise tief in den Wundraum und weiter vorzugsweise bis zum Wundgrund gelangt und dort ihre intendierte Wirkung ausüben kann. Die zugeführte Flüssigkeit und gegebenenfalls zusätzliche Wundflüssigkeiten oder Wundsekrete werden in an sich bekannter Weise über die Saugleitung abgeführt und in Richtung auf eine Unterdruck erzeugende Einrichtung gesogen. Vorrichtungen zur Bereitstellung von Unterdruck für die Unterdruckbehandlung von Wunden, die neben einer Unterdruck erzeugenden Einrichtung auch einen nach Gebrauch vorzugsweise wegwerfbaren Einweg-Behälter zur Aufnahme von Körperflüssigkeitenaufweisen, sind insbesondere in der DE 10 2009 038 130 A1 und DE 10 2009 038 131 A1 der Anmelderin vorbeschrieben. Eine derartige Vorrichtung weist einen Anschluss für eine wie hier in Rede stehende zum Körper eines Patienten führende Saugleitung auf. Die Merkmale dieser vorbekannten Vorrichtungen seien durch Bezugnahme auf die vorgenannten Anmeldungen auch in diese Anmeldung einbezogen. Die erfindungsgemäß vorgesehene Fluidzuführleitung und eine hiermit zusammenwirkende Fluidzuführvorrichtung kann entweder losgelöst und separat von einer Vorrichtung zur Bereitstellung von Unterdruck für medizinische Anwendungen verwirklicht werden, etwa in Form einer Instillationseinrichtung, oder die Fluidzuführvorrichtung kann in die genannte Vorrichtung zur Bereitstellung von Unterdruck integriert sein.

Nach einer bevorzugten Ausbildung der erfindungsgemäßen Anschlussvorrichtung erweist es sich als vorteilhaft, wenn die Fluidzuführleitung zumindest im Bereich der Anschlussvorrichtung parallel zu der Saugleitung geführt ist. Dies vereinfacht die Anbringung auf dem Unterdruckverband, aber auch die Anordnung und Führung der Leitungsmittel gegenüber dem Patienten. Dessen ungeachtet erweist sich eine parallele Führung der Leitungsmittel im Bereich des Trägermittels als zweckmäßig.

Es sei an diester Stelle darauf hingewiesen, dass das flächenhafte unterdruckdichte und biegsame Trägermittel für die Saugleitung und in der Folge auch für die Fluidzuführleitung eine flächenhaft ausgebildete Schnittstelle zwischen der Saugleitung und dem Unterdruckverband bezeichnet. Das Trägermittel bildet zusammen mit der Saugleitung und der weiter herangeführten Fluidzuführleitung einen flachen, biegsamen und flächenhaft anmutenden Aufbau. Das Trägermittel ist jedenfalls in der Draufsicht flächenhaft ausladender ausgebildet als die daran anschließende Saugleitung und die zusätzlich herangeführte Fluidzuführleitung. Somit bildet das Trägermittel einen größeren Anschlussbereich gegen die wundabgewandte Oberseite des Unterdruckverbands.

Weitere Merkmale, Einzelheiten und Vorteile der Erfindung ergeben sich aus den beigefügten Patentansprüchen und aus der nachfolgenden Beschreibung einer bevorzugten Ausführungsform einer erfindungsgemäßen Wundauflage und ihrer Anwendung bei der Unterdruckbehandlung von Wunden. In der Zeichnung zeigt:
Figur 1 eine perspektivische nicht maßstabsgetreue Ansicht einer Anschlussvorrichtung zur Verwendung bei der Unterdruckbehandlung von Wunden, sowie eine angedeutete Wunde mit einem Unterdruckwundverband; und
Figur 2 eine schematische nicht maßstabsgetreue Schnittansicht der Anschlussvorrichtung nach Figur 1 mit Schnittebene II-II;
Figur 3 eine schematische nicht maßstabsgetreue Schnittansicht der Anschlussvorrichtung nach Figur 1 mit Schnittebene III-III;
Figur 4 eine schematische nicht maßstabsgetreue Schnittansicht der Anschlussvorrichtung im Zusammenwirken mit einer erfindungsgemäßen Wundauflage;
Figur 5 schematische Darstellungen von zur Erfindung gehörenden Wundauflagen.

Die Figuren zeigen verschiedene Ansichten einer insgesamt mit dem Bezugszeichen 2 bezeichneten Anschlussvorrichtung zur Verwendung bei der Unterdruckbehandlung von Wunden. Die dargestellte Anschlussvorriclitung 2 wird auf eine wundabgewandte Oberseite 4 eines schematisch Unterdruckverbands 6, welcher eine zu behandelnde Wunde 8 überfängt und zur Atmosphäre hin unterdruckdicht abschließt, aufgebracht und daran auf noch zu beschreibende weise klebend befestigt.

Die Anschlussvorrichtung 2 umfasst ein beispielhaft dargestelltes flaches Leitungsmittel in Form einer Saugleitung 10 aus einem elastomeren biegsamen Material und ein flächenhaft erstrecktes, plattenförmiges Trägermittel 12, welches die flache Saugleitung 10 hält und stützt, so dass auf die Saugleitung 10 ausgeübte Druck- oder Biegekräfte gleichmäßig in das flächenhafte Trägermittel 12 eingeleitet und von diesem aufgenommen werden können.

Die Saugleitung 10 ist derart flachbauend ausgebildet, dass sie in einem Längsendabschnitt 14 mit vorzugsweise nahezu 100 % ihrer senkrecht auf das Trägermittel 12 projizierten Fläche mit dem Trägermittel 12 verbunden ist. Die Saugleitung 10 kann mit ihrer betreffenden Flachseite 15 auf die wundabgewandte Oberseite 16 des Trägermittels 12 aufgeklebt oder aufvulkanisiert, also thermisch gefügt sein.

Vorzugsweise nach dem unterdruckdichten Fügen der Saugleitung 10 mit dem Trägermittel 12 werden durch das Trägermittel 12 und durch die Saugleitung 10 hindurchgehende Öffnungen 18 ausgebildet, die mit einer schematisch dargestellten Öffnung 20 oder mehreren Öffnungen 20 in dem Unterdruckverband 6 kommunizieren, wenn die Anschlussvorrichtung 2 auf den Unterdruckverband 6 appliziert ist, um einen Unterdruck in den Wundraum anzulegen.

Die Dickenerstreckung D des Verbunds aus Saugleitung 10 und Trägermittel 12 beträgt höchstens 7 mm, vorzugsweise höchstens 5 mm und weiter vorzugsweise lediglich 3 - 4 mm.

Aus den Figuren erkennt man weiter, dass die Anschlussvorrichtung 2 auf der wundzugewandten Seite 22 des Trägermittels 12 eine Haftvermittlungsschicht 24 aufweist. Mittels dieser Haftvermittlungsschicht 24 ist die Anschlussvorrichtung 2 gegen die wundabgewandte Oberseite 4 des Unterdruckverbands 6 klebend haftend aufbringbar. Die Haftvermittlungsschicht 24 ist dreilagig ausgebildet und umfasst eine mittlere Traglage 26, eine an der Traglage 26 gehaltene und dem Trägermittel 12 zugewandte erste Kleberschicht 28 und eine an der Traglage 26 gehaltene und von dem Trägermittel 12 abgewandte, d. h. dem Unterdruckverband 6 zugewandte zweite Kleberschicht 30. Im beispielhaft und bevorzugt dargestellten Fall sind alle drei Lagen der Haftvermittlungsschicht 24 deckungsgleich miteinander ausgebildet. Die Haftvermittlungsschicht 24 ist als Ganzes von einem beidseitig mit Klebermaterial beschichteten Flachmaterial abgelängt bzw. ausgestanzt. Sie ist im beispielhaft dargestellten Fall rahmenförmig, also in Umfangsrichtung durchgehend ausgebildet und erstreckt sich um denjenigen zentralen Bereich 32 des Trägermittels 12 herum, in dem die schematisch dargestellten Öffnungen 18 des Trägermittels 12 bzw. der Saugleitung 10 in Richtung auf den Unterdruckverband 6 ausmünden. Die klebende Fläche der ersten und zweiten Kleberschichten 28, 30 beträgt im beispielhaft dargestellten Fall nur ca. 30 cm². Es sei an dieser Stelle aber auch ausdrücklich erwähnt, dass sich die Haftvermittlungsschicht 24 auch über die gesamte wundzugewandte Seite 22 des Trägermittel 12 erstrecken kann, wobei sie solchermaßen mit den Öffnungen 18 in dem Trägermittel 12 bzw. in der Saugleitung 10 fluchtende oder kommunizierende Durchtrittsöffnungen aufweisen muss. Die klebende Fläche beträgt dann gleichwohl nur ca. 40 cm².

Die Kleberschichten 28 und 30 sind von unterschiedlichen Klebematerialien gebildet, die entsprechend auf die Materialien des Trägermittels 12 bzw. des Unterdruckverbands 6 optimiert sind. Sofern beispielsweise das Trägermittel 12 aus Silikon gebildet ist, empfiehlt es sich, dass die erste Kleberschicht 28 einen Silikonkleber umfasst. Wenn derjenige Unterdruckverband 6, für den die Anschlussvorrichtung 2 konzipiert ist, aus Polyurethan bzw. mit einer wundabgewandten Oberseite 4 aus Polyurethan ausgebildet ist, so empfiehlt es sich, dass die zweite Kleberschicht 30 einen Acrylatkleber umfasst, der besonders gut auf Polyurethan haftet. Durch die Verwendung der erfindungsgemäßen Haftvermittlungsschicht 24 können jedenfalls deren beide Kleberschichten 28 und 30 optimal auf die mit ihnen haftend zusammenwirkenden Materialien des Trägermittels 12 bzw. des Unterdruckverbands 6 abgestimmt und herstellerseitig in ihrer Zusammensetzung ausgebildet werden.

Die zweite Kleberschicht 30 ist zudem von einer zweiteiligen ablösbaren Schutzschicht 34 überfangen, die eine Anfasslasche 36 zum Ablösen der beiden Teile aufweist.

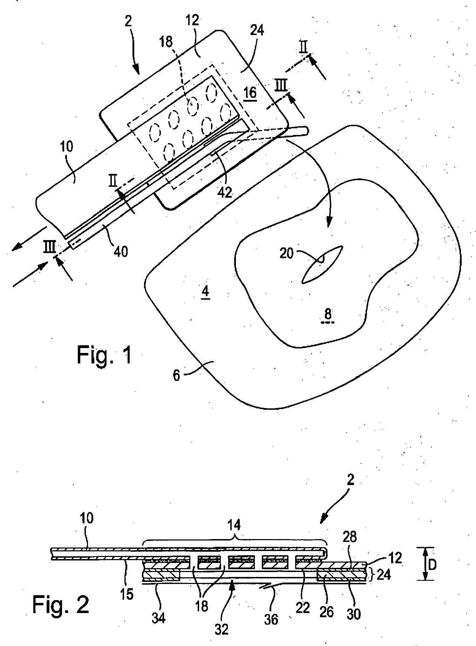

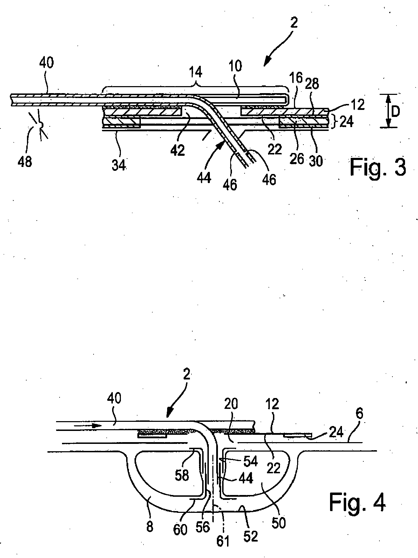

Man erkennt in den Figuren 1 und 3 des weiteren eine Fluidzuführleitung 40, mit der eines Fluids, beispielsweise zur Wundspülung oder zur Instillation der Wunde dem Wundraum zugeführt werden kann. Diese Fluidzuführleitung 40 ist vorzugsweise in derselben Ebene wie die Saugleitung 10 und parallel zu der Saugleitung 10 an das Trägermittel 12 herangeführt; sie ist ebenfalls von dem Trägermittel 12 gehalten und an die wundabgewandte Oberseite 16 des Trägermittels 12, beispielsweise mittels Kleber oder stoffschlüssig unlösbar angefügt. Erfindungsgemäß erstreckt sich die Fluidzuführleitung 40 durch eine weitere Öffnung 42 in dem Trägermittel 12 hindurch auf die wundzugewandte Seite 22 des Trägermittels 12 und steht dort in einem spitzen Winkel in Richtung auf die Wunde vor. Da die Fluidzuführleitung 40 aus einem biegsamen Material der eingangs beschriebenen Art gebildet ist, lässt sich ein vorstehender Endabschnitt 44 der Fluidzuführleitung 40 leicht und im wesentlichen widerstandslos in andere gewünschte Richtungen abbiegen und auf noch zu beschreibende Weise bezüglich der Wunde bzw. einer Wundauflage orientieren. In dem ausragenden Endabschnitt 44 der Fluidzuführleitung 40 können für bestimmte Anwendungen zusätzliche Queröffnungen 46 in der Wandung ausgebildet sein, was lediglich schematisch angedeutet ist. Des Weiteren ist schematisch eine Abklemmeinrichtung 48 angedeutet, mittels der der Querschnitt der Fluidzuführleitung 40 auf Null reduziert werden kann.

Figur 4 zeigt stark schematisiert die Anwendung der Anschlussvorrichtung 2 zusammen mit einer erfindungsgemäßen Wundauflage 50 bei einer Wunde 8, die mit einem Unterdruckverband 6 bis auf die Öffnung 20 abgedichtet ist. Im Bereich dieser Öffnung 20 in dem Unterdruckverband 6 ist die Anschlussvorrichtung 2 insbesondere mittels der erwähnten Haftvermittlungsschicht 24 dichtend aufgebracht. Alternativ könnte die Anschlussvorrichtung 2 mittels zusätzlicher die Anschlussvorrichtung 2 überfangender Klebefolien oder dergleichen dichtend an der wundabgewandten Oberseite 16 des Trägermittels 12 fixiert werden. In der schematisierten Schnittdarstellung der Figur 4 ist nur die Schnittebene durch die Fluidzuführleitung 40 erfasst. Man erkennt, dass der von der wundzugewandten Seite 22 des Trägermittels 12 vorstehende Endabschnitt 44 der Fluidzuführleitung 40 in die Wundauflage 50 in Richtung auf den Wundgrund 52 eingeführt ist. Hierfür ist in der Wundauflage 50 erfindungsgemäß eine Durchgangsöffnung 54 ausgebildet. In der Durchgangsöffnung 54 ist eine Hülse 56 angeordnet. Die Hülse 56 ist zweiteilig in Form zweier von jeweils gegenüberliegenden Seiten der Wundauflage 50 in die Durchgangsöffnung 54 eingeführter Hülsenteile 58, 60 ausgebildet. Die Hülsenteile 58, 60 sind zueinander teleskopierend in Richtung einer Hülsenlängsachse 61. Auf diese Weise lässt sich die Hülsenlänge entsprechend der Deformation der Wundauflage 50 selbsttätig einstellen. Je nach Unterdruck und Komprimierung der vorzugsweise aus einem absorbierenden Schaum gebildeten Wundauflage 50 kann die Hülsenlänge selbsttätig eingestellt werden. Die Hülse 58 führt den Endabschnitt 44 der Fluidzuführleitung 40 und verhindert, dass dieser gegen den Wundgrund 52 anliegt. Auf diese Weise kann Flüssigkeit über die Fluidzuführleitung 40 an tief gelegene Bereiche der Wunde 8, vorzugsweise bis zum Wundgrund 52 zugeführt werden. Zugeführte Flüssigkeit kann dann zusammen mit Wundflüssigkeiten und Wundsekreten über die in Figur 4 nicht dargestellte Saugleitung 10 abgeführt werden.

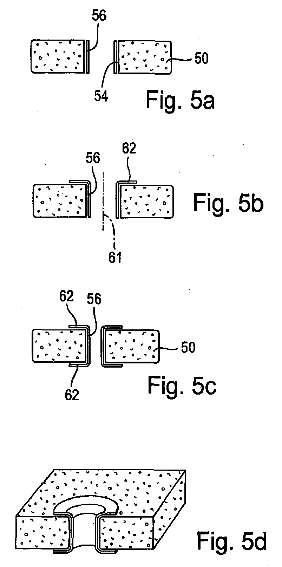

Die Figuren 5a bis f zeigen verschiedene rein schematische Darstellungen erfindungsgemäßer Wundauflagen 50, deren Geometrie nur vereinfacht und schematisch zu verstehen ist. Die jeweiligen Wundauflagen 50 sind aus einem flexibel nachgiebigen Schaummaterial, vorzugsweise der oben im Einzelnen beschriebenen Art, gebildet. Bei der Ausführungsform nach Figur 5a ist die Hülse 56 zylindrisch ausgebildet und in die Durchgangsöffnung 54 der Wundauflage 50 eingesetzt. Sie kann beispielsweise reibschlüssig in der Durchgangsöffnung 54 in unmittelbarem Kontakt zum Schaummaterial der Wundauflage 50 gehalten sein.

Bei der Wundauflage nach Figur 5b weist die Hülse 56 an einem Ende einen krempenförmigen Bundabschnitt 62 auf, so wie dies auch bei den Hülsenteilen 58, 60 gemäß Figur 4 der Fall ist, der bezüglich der Hülsenlängsachse 61 eine axiale Anlage an das Schaummaterial bildet. Der Bundabschnitt 62 ist vorzugsweise einstückig an den übrigen zylindrischen Teil der Hülse 56 angefügt.

Bei der Ausführungsform gemäß Figur 5c weist die Hülse 56 jeweils an beiden Enden einen solchen Bundabschnitt 62 auf. Hierdurch wird zwischen den Bundabschnitten 62 ein Aufnahmeraum für das Schaummaterial der Wundauflage 50 geschaffen, so dass die Hülse 56 gewissermaßen formschlüssig an der Wundauflage 50 gehalten ist. Figur 5d zeigt dies in perspektivischer Ansicht.

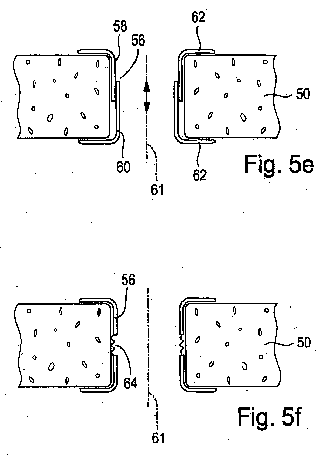

Bei der Ausführungsform gemäß Figur 5e ist eine Hülse 56 aus zwei Hülsenteilen 58, 60 (wie bei Figur 4) gebildet, die jeweils einen Bundabschnitt 62 aufweisen und gegeneinander in Richtung der Hülsenlängsachse 61 gegeneinander teleskopierend verschieblich sind. Die Hülsenteile 58, 60 können sich daher der Deformation der Wundauflage 50 selbsttätig anpassen. Eine andere Ausführungsform zeigt Figur 5f, bei der zwei Hülsenteile 58, 60 über eine faltenbalgartige Kopplung 64 in Richtung der Hülsenlängsachse 61 gegeneinander bewegbar sind.

## Patentansprüche

1. Sachgesamtheit aus einer Wundauflage (50) und aus einem einen Wundraum. im wesentlichen unterdruckdicht abschließenden Unterdruckverband (6) und aus einer Anschlussvorrichtung (2), die eine mit Unterdruck beaufschlagbare Saugleitung (10) und ein flächenhaftes unterdruckdichtes und biegsames Trägermittel (12) für die Saugleitung (10) umfasst, wobei das Trägermittel (12) auf eine wundabgewandte Außenseite des die Wunde überfangenden und gegen die Atmosphäre dichtend abschließenden Unterdruckverbands (6) im wesentlichen unterdruckdicht aufbringbar ist, so dass die Saugleitung (10) durch wenigstens eine Öffnung (18, 20) in dem Trägermittel (12) und in dem Unterdruckverband (6) hindurch mit dem Wundraum kommunizieren kann, und wobei die Wundauflage in einem Wundraum unterhalb des den Wundraum gegen die Atmosphäre im wesentlichen unterdruckdicht abschließenden Unterdruckverbands (6) bestimmungsgemäß anordenbar ist, wobei die Wundauflage (50) ein flexibel nachgiebiges Schaummaterial umfasst, **dadurch gekennzeichnet, dass** eine in derselben Ebene wie die Saugleitung (10) an das Trägermittel (12) herangeführte Fluidzuführleitung (40) zur Zufuhr eines fluiden Mediums zum Wundraum vorgesehen ist, und dass deren Dicke nicht größer als die Dicke der Saugleitung (10) ist, und dass die Fluidzuführleitung (40) durch eine Öffnung in dem Trägermittel (12) dichtend hindurchgeführt ist und von der wundzugewandten Seite (22) des Trägermittels (12) vorsteht und durch die Öffnung (20) in dem Unterdruckverband (6) hindurch in die Wundauflage (50) einragen kann und dass die Wundauflage (50) hierfür eine in Wundtiefenrichtung erstreckte Durchgangsöffnung (54) aufweist, die zum Einführen oder Hindurchführen eines wundseitigen Endabschnitts (44) der Fluidzuführleitung (40) dient, und dass in der Durchgangsöffnung (54) eine Hülse (56) angeordnet ist, in welche dieser Endabschnitt (44) einsteckbar ist, und dass die Länge der Hülse (56) veränderbar ist und sich die Hülse so der Kompression oder Kontraktion des Schaummaterials anpasst.

2. Sachgesamtheit nach Anspruch 1, **dadurch gekennzeichnet, dass** die Hülse (56) direkt gegen das Schaummaterial der Wundauflage anliegt.

3. Sachgesamtheit nach Anspruch 1 oder 2, **dadurch gekennzeichnet, dass** die Hülse (56) wenigstens auf einer Seite, vorzugsweise auf beiden Seiten des Schaummaterials einen Bundabschnitt (62) aufweist, mit dem die Hülse (56) bezüglich einer Hülsenlängsachse (61) axial gegen eine die Öffnung (54) umgebende Seite des Schaummaterials anliegt.

4. Sachgesamtheit nach Anspruch 3, **dadurch gekennzeichnet, dass** der Bundabschnitt (62) in Umfangsrichtung durchgehend geschlossen ausgebildet ist.

5. Sachgesamtheit nach einem oder mehreren der vorstehenden Ansprüche,
**dadurch gekennzeichnet, dass** die Hülse (56) zwei gegeneinander in Richtung der Hülsenlängsachse (61) gleitverschiebliche, teleskopierende Hülsenabschnitte (58, 60) umfasst.

6. Sachgesamtheit nach einem oder mehreren der vorstehenden Ansprüche,
**dadurch gekennzeichnet, dass** die Hülse (56) zwei über eine faltenbalgartige Kopplung (64) in Richtung der Hülsenlängsachse (61) gegeneinander bewegbare Hülsenabschnitte (58, 60) umfasst.

7. Sachgesamtheit nach einem oder mehreren der vorstehenden Ansprüche,
**dadurch gekennzeichnet, dass** das flexibel nachgiebige Schaummaterial einen offenzelligen Polyurethanschaumstoff umfasst oder hieraus gebildet ist, erhältlich durch Umsetzung einer Mischung, umfassend die Komponenten: (i) Polyisocyanat, insbesondere MDI, PMDI und/oder TDI, (ii) Polyol, insbesondere Polyesterpolyol, (iii) Treibmittel, und (iv) Katalysator, und dass der offenzellige Schaumstoff nach dreitägiger Lagerung in Rinderserum eine Zugfestigkeit, gemessen gemäß DIN 53571, zwischen 80 kPa und 300 kPa, insbesondere zwischen 150 kPa und 220 kPa aufweist.

8. Sachgesamtheit nach Anspruch 7, **dadurch gekennzeichnet, dass** als Polyol (ii) Polyesterpolyol verwendet ist, welches erhältlich ist durch Umsetzung einer Dicarbonsäure mit 4 bis 8 Kohlenstoffatomen mit einen Dialkohol mit 2 bis 6 Kohlenstoffatomen.

9. Sachgesamtheit nach Anspruch 7 oder 8, **dadurch gekennzeichnet, dass** der offenzellige Polyurethanschaumstoff eine Bruchdehnung von 250 % bis 650 %, insbesondere 260 bis 320 %, gemessen gemäß DIN 53571, aufweist.

10. Sachgesamtheitnach Anspruch 7, 8 oder 9, **dadurch gekennzeichnet, dass** der offenzellige Polyurethanschaumstoff eine Luftdurchlässigkeit von 1000 bis 8000 I/(m²sec), insbesondere von 2400 bis 3300 I/(m²sec), gemessen gemäß DIN EN ISO 9237, aufweist.

11. Sachgesamtheit nach Anspruch 7, 8, 9 oder 10, **dadurch gekennzeichnet, dass** der offenzellige Polyurethanschaumstoff eine Rohdichte zwischen 15 und 30 kg/m³, insbesondere zwischen 24 und 28 kg/m³ aufweist, gemessen gemäß DIN EN ISO 845.

12. Sachgesamtheit nach Anspruch 7, 8, 9, 10 oder 11, **dadurch gekennzeichnet, dass** der offenzellige Polyurethanschaumstoff einen Aromatenanteil von 5 bis 50 %, insbesondere von 15 bis 40 % aufweist

13. Sachgesamtheit nach einem oder mehreren der vorstehenden Ansprüche,
**dadurch gekennzeichnet, dass** die Saugleitung (10) biegsam und flach ausgebildet ist und in einem wundseitigen Längsendabschnitt (14) mit wenigstens 70 % ihrer senkrecht auf das Trägermittel (12) projizierten Fläche mit dem Trägermittel (12) für den bestimmungsgemäßen Gebrauch unlösbar und flächenhaft verbunden ist, wobei das Trägermittel (12) und die Saugleitung (10) mit ihren aus ihrer flachen Form resultierenden Hauptebenen zueinander parallel sind, und wobei die Dickenerstreckung (D) des Verbunds aus Saugleitung (10) und Trägermittel (12) höchstens 7 mm beträgt.

14. Sachgesamtheit nacheinem oder mehreren der vorstehenden Ansprüche ,
**dadurch gekennzeichnet, dass** auf der wundzugewandten Seite (22) des Trägermittels (12) eine wenigstens dreitägiger Haftvermittlungsschicht (24) vorgesehen ist, die eine mittlere Traglage (26), eine an der Traglage (26) gehaltene und dem Trägermittel (12) zugewandte erste Kleberschicht (28) und eine an der Traglage (26) gehaltene und von dem Trägermittel (12) abgewandte zweite Kleberschicht (30) umfasst und die so ausgebildet ist, dass sie die wenigstens eine Öffnung (18) in dem Trägermittel (12) nicht blockiert.

## Claims

1. A combination comprised of a wound dressing (50) and of a negative-pressure bandage (6) that closes off a wound space in essentially vacuum-tight fashion, and of a connection device (2) that includes a suction line (10), which can be subjected to negative pressure, and a two-dimensional, vacuum-tight and pliable support means (12) for the suction line (10), wherein the support means (12) can be applied in essentially vacuum-tight fashion to an outer side, facing away from the wound, of the negative-pressure bandage (6) that overlies the wound and seals off the wound from the atmosphere, so that the suction line (10) can communicate with the wound space through at least one opening (18, 20) in the support means (12) and in the negative-pressure bandage (6), and wherein the wound dressing can be located as intended in a wound space underneath the negative-pressure bandage (6) that seals off the wound space from the atmosphere in essentially vacuum-tight fashion, and wherein the wound dressing (50) includes a flexibly resilient foam material, **characterized in that** a fluid delivery line (40), carried brought to the support means (12) in the same plane as the suction line (10), is provided for delivering a fluid medium to the wound space; and that its thickness is not greater than the thickness of the suction line (10); and that the fluid delivery line (40) is passed sealingly through an opening in the support means (12) and protrudes from the side (22) of the support means (12) facing toward the wound and can protrude into the wound dressing (50) through the opening (20) in the negative-pressure bandage (6); and that for that purpose the wound dressing (50) has a through opening (54), extending in the depthwise direction of the wound, through which an end portion (44), toward the wound, of the fluid delivery line (40) can be introduced or passed; and that located in the through opening (54) is a sheath (56) into which this end portion (44) can be inserted; and that the length of the sheath (56) is variable, and the sheath in this way adapts to the compression or contraction of the foam material.

2. The combination of claim 1, **characterized in that** the sheath (56) is in direct contact with the foam material of the wound dressing.

3. The combination of claim 1 or 2, **characterized in that** the sheath (56), on at least one side and preferably on both sides of the foam material, has a collar portion (62), with which the sheath (56) is in axial contact axially, relative to a longitudinal axis (61) of the sheath, against a side of the foam material surrounding the opening (54).

4. The combination of claim 3, **characterized in that** the collar portion (62) is embodied as continuously closed in the circumferential direction.

5. The combination of one or more of the foregoing claims, **characterized in that** the sheath (56) includes two telescoping sheath portions (58, 60) that are slidably displaceable counter to one another in the direction of the longitudinal axis (61) of the sheath.

6. The combination of one or more of the foregoing claims, **characterized in that** the sheath (56) includes two sheath portions (58, 60) that are movable counter to one another in the direction of the longitudinal axis (61) of the sheath via a bellowslike coupling (64).

7. The combination of one or more of the foregoing claims, **characterized in that** the flexibly resilient foam material includes or is formed of an open-pore polyurethane foam, the polyurethane foam being obtainable by reacting a mixture including the following components:
(i) polyisocyanate, in particular MDI, PMDI and/or TDI,
(ii) polyol, in particular polyester polyol, (iii) propellant, and (iv) catalyst; and that the open-pore foam, after storage for three days in cow serum, has a tensile strength, measured per DIN 53571, of between 80 kPa and 300 kPa, in particular between 150 kPa and 220 kPa.

8. The combination of claim 7, **characterized in that** as the polyol (ii), polyester polyol is used, which is obtainable by reacting a dicarboxylic acid that has from 4 to 8 carbon atoms with a dialcohol that has from 2 to 6 carbon atoms.

9. The combination of claim 7 or 8, **characterized in that** the open-pore polyurethane foam has a maximum elongation of 250% to 650%, in particular 260 to 320%, measured per DIN 53571.

10. The combination of claim 7, 8 or 9, **characterized in that** the open-pore polyurethane foam has an air permeability of 1000 to 8000 1/(m²sec), in particular of 2400 to 3300 1/(m²sec), measured per DIN EN ISO 9237.

11. The combination of claim 7, 8, 9 or 10, **characterized in that** the open-pore polyurethane foam has a raw density of between 15 and 30 kg/m³, in particular between 24 and 28 kg/m³, measured per DIN EN ISO 845.

12. The combination of claim 7, 8, 9, 10 or 11, **characterized in that** the open-pore polyurethane foam has an aromatic proportion of 5 to 50%, in particular of 15 to 40%.

13. The combination of one or more of the foregoing claims, **characterized in that** the suction line (10) is embodied as pliable and flat and for its intended use is joined, in a longitudinal end portion (14) toward the wound, nondetachably and two-dimensionally to the support means (12) by at least 70% of its area projected vertically onto the support means (12), and the support means (12) and the suction line (10) are parallel to one another with their main planes that result from their flat shape, and wherein the combined thickness (D) of the suction line (10) and support means (12) is at most 7 mm.

14. The combination of one or more of the foregoing claims, **characterized in that** on the side (22) of the support means (12) toward the wound, an at least three-ply adhesion-promoting layer (24) is provided, which includes a middle supporting ply (26), a first adhesive ply (28) retained on the supporting ply (26) and facing toward the support means (12), and a second adhesive ply (30), retained on the supporting ply (26) and facing away from the support means (12), and which adhesion-promoting layer is embodied such that it does not block the at least one opening (18) in the support means (12).

## Revendications

1. Ensemble matériel se composant d'un pansement pour plaie (50) et d'un pansement à pression négative (6) fermant une zone de plaie d'une manière pour l'essentiel étanche à la pression négative ainsi que d'un dispositif de raccordement (2) qui comprend une conduite d'aspiration (10) pouvant être mise en pression négative et un moyen support (12) en nappe pour la conduite d'aspiration (10), qui est étanche à la pression négative et souple, dans lequel ledit moyen support (12) peut être appliqué, d'une manière pour l'essentiel étanche à la pression négative, sur une face extérieure opposée à la plaie du pansement à pression négative (6) recouvrant la plaie et fermant celle-ci de manière étanche par rapport à l'atmosphère, de sorte que la conduite d'aspiration (10) peut communiquer avec la zone de la plaie à travers au moins une ouverture (18, 20) présente dans le moyen support (12) et dans le pansement à pression négative (6), et dans lequel le pansement pour plaie peut être disposé de manière conforme dans une zone de plaie au-dessous du pansement à pression négative (6) fermant la zone de plaie par rapport à l'atmosphère d'une manière pour l'essentiel étanche à la pression négative, dans lequel le pansement pour plaie (50) comprend une matière de mousse flexiblement élastique, **caractérisé par le fait qu'**une conduite d'alimentation en fluide (40) qui est menée au moyen support (12) dans le même plan que la conduite d'aspiration (10) et est destinée à amener un milieu fluide à la zone de la plaie est prévue et que l'épaisseur de celle-ci n'est pas supérieure à l'épaisseur de la conduite d'aspiration (10), et que la conduite d'alimentation en fluide (40) passe de manière étanche à travers une ouverture présente dans le moyen support (12) et fait saillie de la face (22) du moyen support (12) laquelle montre vers la plaie, et peut se projeter dans le pansement pour plaie (50) à travers l'ouverture (20) présente dans le pansement à pression négative (6) et que, à cette fin, le pansement pour plaie (50) présente une ouverture de passage (54) qui s'étend dans la direction de profondeur de plaie et qui sert à introduire ou à faire passer une portion d'extrémité (44) côté plaie de la conduite d'alimentation en fluide (40), et que dans ladite ouverture de passage (54) est disposée une douille (56) dans laquelle cette portion d'extrémité (44) peut être engagée, et que la longueur de la douille (56) est modifiable et que, ainsi, la douille s'adapte à la compression ou contraction de la matière de mousse.

2. Ensemble matériel selon la revendication 1, **caractérisé par le fait que** la douille (56) s'applique directement contre la matière de mousse du pansement pour plaie.

3. Ensemble matériel selon la revendication 1 ou 2, **caractérisé par le fait que** la douille (56) présente, sur au moins une face, de préférence sur les deux faces de la matière de mousse, une portion de collet (62) par laquelle la douille (56) s'applique axialement par rapport à un axe longitudinal de douille (61) contre une face de la matière de mousse, qui entoure l'ouverture (54).

4. Ensemble matériel selon la revendication 3, **caractérisé par le fait que** ladite portion de collet (62) est réalisée de manière à être fermée continûment dans la direction circonférentielle.

5. Ensemble matériel selon l'une ou plusieurs des revendications précédentes, **caractérisé par le fait que** la douille (56) comprend deux portions de douille (58, 60) déplaçables en glissement l'une contre l'autre dans la direction de l'axe longitudinal de douille (61) et se télescopant.

6. Ensemble matériel selon l'une ou plusieurs des revendications précédentes, **caractérisé par le fait que** la douille (56) comprend deux portions de douille (58, 60) qui peuvent être déplacées l'une contre l'autre dans la direction de l'axe longitudinal de douille (61) par l'intermédiaire d'un accouplement (64) de type soufflet.

7. Ensemble matériel selon l'une ou plusieurs des revendications précédentes, **caractérisé par le fait que** la matière de mousse flexiblement élastique comprend une mousse de polyuréthane à cellules ouvertes ou est réalisée à partir de celle-ci, apte à être obtenue par mise en réaction d'un mélange comprenant les composants: (i) le polyisocyanate, en particulier le MDI, le PMDI et/ou le TDI, (ii) le polyol, en particulier le polyol de polyester, (iii) de l'agent de gonflement et (iv) du catalyseur, et que, après un stockage de trois jours dans du sérum bovin, la mousse à cellules ouvertes présente une résistance à la traction, mesurée selon DIN 53571, comprise entre 80 kPa et 300 kPa, en particulier entre 150 kPa et 220 kPa.

8. Ensemble matériel selon la revendication 7, **caractérisé par le fait que** l'on utilise, en tant que polyol, (ii) le polyol de polyester qui peut être obtenu par mise en réaction d'un acide dicarboxylique ayant 4 à 8 atomes de carbone avec un dialcool ayant 2 à 6 atomes de carbone.

9. Ensemble matériel selon la revendication 7 ou 8, **caractérisé par le fait que** la mousse de polyuréthane à cellules ouvertes présente un allongement à la rupture compris entre 250 % et 650 %, en particulier 260 et 320 %, mesuré selon DIN 53571.

10. Ensemble matériel selon la revendication 7, 8 ou 9, **caractérisé par le fait que** la mousse de polyuréthane à cellules ouvertes présente une perméabilité à l'air de 1000 à 8000 1/(m²sec), en particulier de 2400 à 3300 l/(m²sec), mesurée selon DIN EN ISO 9237.

11. Ensemble matériel selon la revendication 7, 8, 9 ou 10, **caractérisé par le fait que** la mousse de polyuréthane à cellules ouvertes présente une densité apparente comprise entre 15 et 30 kg/m³, en particulier entre 24 et 28 kg/m³, mesurée selon DIN EN ISO 845.

12. Ensemble matériel selon la revendication 7, 8, 9, 10 ou 11, **caractérisé par le fait que** la mousse de polyuréthane à cellules ouvertes présente une proportion d'aromatique de 5 à 50 %, en particulier de 15 à 40 %.

13. Ensemble matériel selon l'une ou plusieurs des revendications précédentes, **caractérisé par le fait que** la conduite d'aspiration (10) est réalisée de manière souple et plate et est reliée au moyen support (12) dans une portion terminale longitudinale (14) côté plaie, par au moins 70 % de sa surface projetée verticalement sur le moyen support (12), de manière inamovible et en nappe pour l'utilisation conforme à la destination, dans lequel ledit moyen support (12) et la conduite d'aspiration (10) sont parallèles entre eux avec leurs plans principaux résultant de leur forme plate, et dans lequel l'extension en épaisseur (D) de l'assemblage se composant de la conduite d'aspiration (10) et du moyen support (12) est de 7 mm tout au plus.

14. Ensemble matériel selon l'une ou plusieurs des revendications précédentes, **caractérisé par le fait que** sur la face (22) du moyen support (12), qui montre vers la plaie, est prévue une couche d'agent adhésif (24) ayant au moins trois couches qui comprend une couche porteuse centrale (26), une première couches de colle (28) maintenue sur ladite couche porteuse (26) et montrant vers le moyen support (12) ainsi qu'une deuxième couche de colle (30) maintenue sur la couche porteuse (26) et montrant dans la direction opposée au moyen support (12), et qui est réalisée de manière à ce qu'elle ne bloque pas ladite au moins une ouverture (18) présente dans le moyen support (12).
